# EUROPEAN PATENT SPECIFICATION

(11) **EP 0 971 866 B1**
(45) Date of publication and mention of the grant of the patent: **12.06.2002**
(21) Application number: 98911900.3
(22) Date of filing: 19.03.1998
(51) Int. Cl.: C07C 17/26

(54) **ADDITION OF HYDROFLUOROCARBONS TO FLUOROOLEFINS**
ADDITIONSREAKTION VON HYDROFLUOROKOHLENSTOFFE AN FLUOROOLEFINE
AJOUT D'HYDROFLUOROCARBURES A DES FLUORO-OLEFINES

(30) Priority: 24.03.1997 RU 97106117
(43) Date of publication of application: 19.01.2000
(73) Proprietor: E.I. DU PONT DE NEMOURS AND COMPANY, Wilmington Delaware 19898 (US)
(72) Inventor: BELEN'KII, Gennadii G., Moscow, 11792 (RU); PETROV, Viacheslav A., Hockessin, DE 19707 (US); RESNICK, Paul, R., Cary, NC 27511 (US)
(74) Representative: Jones, Alan John
(86) International application number: PCT/US98/05541
(87) International publication number: WO 98/42645

(56) References cited:
- HASZELDINE ET AL.: "Reaction of hexafluoropropene with halogenoalkanes" JOURNAL OF FLUORINE CHEMISTRY, vol. 21, no. 2, 1982, pages 253-259, XP002067833

## Description

### FIELD OF THE INVENTION

This invention concerns a process for the antimony pentafluoride catalyzed addition of hydrofluorocarbons across the carbon-carbon double bond of fluoroolefins.

### BACKGROUND

Processes for the addition of 1,1,1-trifluoroethane to fluorinated olefins using antimony pentafluoride as a catalyst have been described (see G. G. Belen'kii and L. S. German, Soviet Scientific Reviews, Sect. B, pp. 195-6, M. E. Volpin ed., (Harwood Academic Publishers, 1984). Processes for the addition of trifluoromethanes, including CHF₃, to certain fluorinated olefins using aluminum chlorofluoride as a catalyst have also been described (see U.S. Patent Application No. 06/000,720 and International Application No. PCT/US96/10872). Hydrofluorocarbons can be prepared by both processes. R. N. Hazeldine et al, *J. Fluorine Chem*., 21 (1982) 253-259 discloses a process for the insertion of hexafluoropropene under thermal and/or photochemical conditions into C-H bonds of various halogenomethanes and halogenoethanes. The use of CH₃F to produce CH₂FCF₂CHFCF₃, and of CH₂F₂ to produce CHF₂CF₂CHFCF₃ are illustrated.

Hydrofluorocarbon products are useful as refrigerants, fire extinguishants, heat transfer media, propellants, foaming agents, gaseous dielectrics, sterilant carriers, polymerization media, particulate removal fluids, carrier fluids, buffing abrasive agents, displacement drying agents and power cycle working fluids. There is an interest in developing more efficient processes for the manufacture of hydrofluoroalkanes.

### SUMMARY OF THE INVENTION

A process is provided for forming an adduct of the formula RR¹R²CCR¹R²F or (FR¹R²CCRR²CH₂)₂ wherein R is selected from the group consisting of CH₃, CH₂F, C₂H₄F and F(CF₂)ₙCH₂CH₂ where n is an integer from 1 to 10, each R¹ is independently selected from the group consisting of H, Cl, F and CF₃ and each R² is independently selected from the group consisting of H, F and CF₃. The process comprises reacting a saturated compound of the formula RF with an olefin of the formula R¹R²C=CR¹R² in the liquid phase in the presence of antimony pentafluoride catalyst; provided that when (FR¹R²CCR¹R²CH₂)₂ is formed, the saturated compound is CH₃CHF₂ or CH₂FCH₂F and anhydrous HF is present.

### DETAILED DESCRIPTION OF THE INVENTION

This invention provides a liquid phase process for the addition of (a) saturated compounds of the formula RF, where R is selected from the group consisting of CH₃, CH₂F, C₂H₄F and F(CF₂)ₙCH₂CH₂, where n is an integer from 1 to 10, to (b) olefins of the formula R¹R²C=CR¹R² where each R¹ is independently selected from the group consisting of H, Cl, F and CF₃ and each R² is independently selected from the group consisting of H, F and CF₃; to form adducts of the formula RR¹R²CCR¹R²F or (FR¹R²CCR¹R²CH₂)₂ in the presence of antimony pentatluoride catalyst (provided that when (FR¹R²CCR¹R²CH₂)₂ is formed, the saturated compound is CH₃CHF₂ or CH₂FCH₂F and anhydrous HF is present).

Examples of olefins of the formula R¹R²C=CR¹R² which can be used in the process of this invention include CF₂=CF₂, CF₃CF=CF₂, CClF=CF₂, CClF=CClF, CHF=CF₂, CH₂=CF₂, CF₃CH=CF₂, CHF=CFCF₃ and CH₂=C(CF₃)CF₃. Some of the olefins are commercially available, the others can be prepared by known methods.

Examples of saturated compounds of the formula RF which can be used in the process of this invention include CH₃F, CH₂F₂ and CH₃CHF₂.

Solvents or diluents may be employed in the process of the present invention. The solvent or diluent is selected so that it will not be reactive in the process or lead to the deactivation of the antimony fluoride catalyst. Suitable solvents or diluents may be selected from the group consisting of perfluoroalkanes or perfluoroethers (e.g., perfluorocyclobutane); the cyclic dimer of hexafluoropropene (i.e., the isomeric perfluorodimethylcyclobutanes); perfluoroethers or perfluoro tertiary amines. Preferred on the basis of its ready availability to those skilled in the art is the cyclic dimer of hexafluoropropene.

In one embodiment a one to one adduct of the saturated compound and the olefin is formed. It is noted in this regard that the saturated compounds of the formula F(CF₂)ₙCH₂CH₂F can themselves be produced as one to one adducts. For example, the addition reaction of CH₂FCH₂F to CF₂=CF₂ can be used to produce F(CF₂)₂CH₂CH₂F.

In a second embodiment of this invention the addition of the hydrofluorocarbons CH₃CHF₂ and/or FCH₂CH₂F to olefins of the formula R¹R²C=CR¹R² in the presence of antimony pentafluoride can be done in the presence of anhydrous HF. The molar ratio of HF:SbF₅ is in the range of 10:1 to 40:1, preferably 20:1. The addition product or adduct is (FR¹R²CCR¹R²CH₂)₂. Without wishing to be bound by theory, it is believed that when CH₃CHF₂ is used it isomerizes to FCH₂CH₂F before reaction with the olefin.

When the addition product (adduct) contains chlorine, the chlorine can be removed by either reaction with HF in the presence of a fluorination catalyst (e.g., Cr₂O₃) or by reaction with hydrogen in the presence of a hydrogenation catalyst (e.g., palladium supported on carbon). The adduct is thereby converted from a hydrochlorofluorocarbon to a hydrofluorocarbon.

The temperature employed in the process of the present invention typically ranges from about -10°C to about 100°C. The preferred temperature range is from about 0°C to 80°C.

Reaction time is not critical and typically ranges from about 5 seconds to about 24 hours. From about I to 16 hours, are usually sufficient.

The pressure employed in the reaction is not critical. The reaction is normally run at pressures in the range of from 0 to 300 psig (101 kPa to 2169 kPa). Autogenous pressures are usually employed; however the pressure should not be allowed to rise above 300 psig when using tetrafluoroethylene because of safety considerations.

Where the reaction conditions are heterogeneous, some degree of agitation is often desirable.

Since the catalysts are water sensitive, reagents and equipment should be dried before use.

The proportion of catalyst to the olefin reactant is typically from about 0.01:1 to about 0.5:1; a range of from 0.1:1 to about 0.5:1 is preferred.

The proportion of saturated compound to olefin is preferably at least about preferably 1:1, when forming RR¹R²CCR¹R²F and preferably at least about 2:1 when forming (FR¹R²CCR¹R²CH₂)₂. Of note are embodiments which use saturated compounds as a solvent such that they are present in substantial excess.

The reaction can be done in batch, semi-batch, semi-continuous or continuous modes in one or more reaction vessels. On a laboratory scale, the reaction can be done in shaker tubes, where all reagents are combined before the reaction vessel is sealed and the reaction begun. It can also be done in autoclaves equipped with an agitator. Product(s) may be isolated by standard chemical engineering techniques, e.g., fractional distillation.

Without further elaboration, it is believed that one skilled in the art can, using the description herein, utilize the present invention to its fullest extent. The following specific embodiments are, therefore, to be construed as merely illustrative, and do not constrain the remainder of the disclosure in any way whatsoever.

### EXAMPLES

### EXAMPLE 1

### CH₂F₂ + CF₂=CF₂ → CH₂FCF₂CF₃

Difluoromethane (39.0 g, 0.75 mol), antimony pentafluoride (45 g, 0.2 mol) and tetrafluoroethylene (30.0 g, 0.3 mol) were added to a 250 mL steel autoclave. The reaction mixture was stirred at 50°C for 8 hours, additional tetrafluoroethylene (30,0 g, 0.3 mol) added, and stirring was maintained at 50°C for 8 more hours. The gaseous reaction products were condensed in a trap, then distilled to yield CH₂FCF₂CF₃ (75 g. 80% yield), b.p. 0-1°C.
¹⁹F NMR (δ, ppm, relative to CF₃CO₂H) of CH₂F^{C}CF₂^{B}CF₃^{A}: A 8.5, B 51.5, C 167.6, J_{F}^{A-C} is 9 Hz, J_{F}^{B}_{-H} is 12 Hz, J_{F}^{C}_{-H} is 45 Hz. ¹H NMR (δ, ppm, relative to TMS): 5.26 (CH₂).

### COMPARATIVE EXAMPLE A

### CH₂F₂ + CF₂=CF₂ → No Reaction )

Difluoromethane (26 g), aluminum chlorofluoride (AlClₓF_{y} where x+y=3, 5 g) and tetrafluoroethylene (30 g) were added to a 400 mL Hastelloy™ nickel alloy shaker tube. The aluminum chlorofluoride was prepared by the reaction of AlCl₃ and CCl₃F according to the method described in U.S. Pat. No. 5,162,594, column 4, lines 35-57. The reaction mixture was agitated at 23°C for 12 hours. No reaction was detected.

### EXAMPLE 2

### CH₂F₂ + CF₂ = CFCF₃ → CH₂FCF(CF₃)₂

Difluoromethane (39.0 g, 0.75 mol), antimony pentafluoride (45 g, 0.2 mol) and perfluoropropylene (66 g, 0.44 mol) were added to a 250 mL steel autoclave. The reaction mixture was stirred at 80°C for 20 hours. The reaction products were condensed in a trap, then distilled to yield CH₂FCF(CF₃)₂ (80 g, 90% yield), b.p. 22-24°C.
¹⁹F NMR (δ) of CH₂F^{C}CF^{B}(CF₃^{A})₂: F^{A} 0.5, F^{B} 114, F^{C} 167.5, J_{F}^{A}_{-F}^{B} is 7 Hz, J_{F}^{A}_{-F}^{C} is 9.5 Hz, J_{F}^{C}_{-F}^{B} is 12.5 Hz, J_{F}^{A}_{-H} is 11 Hz. ¹H NMR (δ): 5.25 (CH₂), J_{F}^{C}_{-H} is 45 Hz, J_{F}^{B}-H is 16 Hz.
Mass spectrum (m/z, assignment, %): 201 (M-H)⁺ 0.03, 183 (M-F)⁺ 2.04, 163 (C₄HF₆)⁺ 1.4, 150 (C₃F₆)⁺ 34.4, 133 (C₃H₂F₅)⁺ 3.4, 131 (C₃F₅)⁺ 6.3, 119 (C₂F₅)⁺ 4.4, 114 (C₃H₂F₄)⁺ 229, 113 (C₃HF₄)⁺ 27.3, 100 (C₂F₄)⁺ 22.6, 69 (CF₃)⁺ 100.

### EXAMPLE 3

### CH₃F + CF₂=CF₂ → CH₃CF₂CF₃

Fluoromethane (35.0 g), antimony pentafluoride (15 g) and tetrafluoroethylene (50 g) were added to a 400 mL Hastelloy™ nickel alloy shaker tube. The reaction mixture was agitated at 25°C for 6 hours followed by agitation at 50°C for 10 hours. The reaction products were condensed in a dry-ice trap, then distilled to yield 99% pure CH₃CF₂CF₃ (HFC-245cb, 45 g), b.p. -13 to -12°C. The yield of HFC-245cb was 67%.

### COMPARATIVE EXAMPLE B

### CHF₃ + CF₂=CF₂ → PTFE

Trifluoromethane (21 g), antimony pentafluoride (7 g) and tetrafluoroethylene (30 g) were added to a 400 mL Hastelloy™ nickel alloy shaker tube. The reaction mixture was agitated at 25°C for 12 hours. The only product isolated was polytetrafluoroethylene (PTFE, 15 g).

### EXAMPLE 4

### CH₃F + CF₂=CFCF₃ → (CF₃)₂CHF + (CF₃)₂CFCH₃ + (CF₃)₂CFC(CH₃)₃ + (CF₃)₂CFC₂H₅

Fluoromethane (20 g), antimony pentafluoride (15 g) and hexafluoropropylene (75 g) were added to a 400 mL Hastelloy™ nickel alloy shaker tube. The reaction mixture was agitated at 50°C for 14 hours. The reaction products were condensed in a dry-ice trap, then distilled to yield a mixture (68 g) containing, based on ¹H and ¹⁹F NMR, 66 % (CF₃)₂CHF, 11% (CF₃)₂CFCH₃, 5% (CF₃)₂CFC₂H₅, 9% (CF₃)₂CFC(CH₃)₃. The yield of (CF₃)₂CFCH₃ was 4%.

### EXAMPLE 5

### CH₃F + CClF=CF₂ → ClCF₂CF₂CH₃ + CF₃CF₂CH₃ + CF₃CClFCH₃

Fluoromethane (17 g), antimony pentafluoride (15 g) and chlorotrifluoroethylene (59 g) were added to a 400 mL Hastelloy™ nickel alloy shaker tube. The reaction mixture was agitated at 50°C for 12 hours. The reaction products were condensed in a dry-ice trap, then distilled to yield a mixture (12 g) containing, based on ¹H and ¹⁹F NMR, 87.5% ClCF₂CF₂CH₃, 6.5% CF₃CF₂CH₃, 4% CF₃CFClCH₃ and 1% unidentified product. The yield of chlorofluoropropanes was 17.4%.

### EXAMPLE 6

### CH₃F + CHF=CF₂ → CH₃CHFCF₃ + CH₂FCF₃

Fluoromethane (10 g), antimony pentafluoride (10 g) and trifluoroethylene (24 g) were added to a 400 mL Hastelloy™ nickel alloy shaker tube. The reaction mixture was agitated at 25°C for 14 hours. The reaction products were condensed in a dry-ice trap, then distilled to yield a mixture (12 g, b.p. 0 to 2°C) containing, based on ¹H and ¹⁹F NMR, 90% CH₃CHFCF₃ and 10% CH₂FCF₃. The yield of CH₃CHFCF₃ was 38.6%.

### EXAMPLE 7

### CH₂F₂ + CClF=CClF → CH₂ClCF₂CF₂Cl

Difluoromethane (18 L, 0.7 mol), antimony pentafluoride (22 g, 0.1 mol) and 1,2-dichlorodifluoroethylene (26.6 g, 0.2 mol) were added to a 250 mL steel autoclave. The reaction mixture was stirred at room temperature for 8 hours followed by heating the autoclave in a boiling water bath. The reaction products were condensed in a trap cooled to -78°C. Excess difluoromethane was separated by distillation. The residue was washed with water, dried and distilled to give in 32% yield CH₂ClCF₂CF₂Cl (12 g, b.p. 62°C to 64°C).
¹⁹F NMR (δ) of CH₂ClCF₂^{B}CF₂^{A}Cl: -50 s (2F^{A}): 41.5 t (2F^{B}). ¹H NMR (δ): 3.8; J_{[H-F}^{B}_{]} is 14 Hz.
Mass spectrum (m/z, assignment, %): 184 (M⁺, C₃H₂Cl₂F₄⁺. 0.5); 165 (M-F, C₃H₂Cl₂F₃⁺, 0.3); 149 (M-Cl, C₃H₂ClF₄⁺, 17.7): 99 (C₂H₂ClF₂⁺, 100); 85 (CClF₂⁺, 33); 64 (C₂H₂F₂⁺, 12).

### EXAMPLE 8

### CH₃CHF₂ + CF₂=CF₂ → CH₃CHFCF₂CF₃ + CH₂FCH₂CF₂CF₃

1,1-Difluoroethane (53 g, 0.8 mol), antimony pentafluoride (44 g, 0.2 mol) and tetrafluoroethylene (8.5 L, 0.3 mol) were added to a 250 mL steel autoclave. The reaction mixture was stirred at 40°C to 50°C for 10 hours. The reaction products were bubbled through a gas-washing bottle containing water and condensed in a trap cooled to -78°C. Excess 1,1-difluoroethane was separated by distillation. The residue was washed with water, dried and distilled to yield a mixture in 40% yield (20 g, b.p. 23°C to 27°C) containing CH₃CHFCF₂CF₃ (89%) and CH₂FCH₂CF₂CF₃ (11%).
¹⁹F NMR (δ) of CH₃¹CH²F^{D}CF^{A}F^{B}CF₃^{C}: 7.5 (3F^{A}); 55 (F^{A}, F^{B}, AB-system); 120 (F^{D}); J_{F}^{C}_{-F}^{D} is 10 Hz; J_{F}^{A}_{-F}^{B} is 230 Hz; J_{[F}^{A}_{(F}^{B}_{)-F}^{D}_{]} is 16 Hz; J_{[F}^{A}_{(F}^{B}_{)-H}¹_{]} is 7 Hz; J_{[F}^{A}_{(F}^{B}_{)-H}²_{]} is 17 Hz; J_{[F}^{D}_{-H}²_{]} is 45 Hz. ¹H NMR (δ): 1.7 (H¹); 5.25 (H²); J_{[H}¹_{-H}²_{]} is 7 Hz.
¹⁹F NMR (δ) of CH₂¹F^{C}CH₂²CF₂^{B}CF₃^{A}: 11.5 (3F^{A}); 42.5 (2F^{B}); 147.5 (F^{C}); 55 (F^{B}); J(F^{B}-F^{C}) is 5 Hz; J(F^{B}_{-H}¹) is 18 Hz; J(F^{C}_{-H}²) is 45 Hz; J(F^{C}_{-H}¹) is 28 Hz.

### EXAMPLE 9

### CH₃CHF₂ + CF₂=CF₂ → (CF₃CF₂)₂(CH₂)₂ + CF₃CF=CHCH₂CF₂CF₃

Anhydrous HF (80 mL), 1,1-difluoroethane (11 g, 0.2 mol), antimony pentafluoride (45 g, 0.2 mol) and tetrafluoroethylene (40 g, 0.4 mol) were added to a 250 mL steel autoclave. The reaction mixture was agitated at room temperature for 8 hours followed by heating the autoclave in a boiling water bath. The reaction products were collected in a gas-washing bottle containing water. The organic layer was separated, washed with water, dried and distilled to yield a mixture in 50% yield (27 g, b.p. 46 to 58°C) containing CF₃CF₂CH₂CH₂CF₂CF₃ (70%) and CF₃CF=CHCH₂CF₂CF₃ (30%). The reaction mixture also contained a small amount of 1-perfluoroethyl-2,2,3,3-tetrafluorocyclobutane.
¹⁹F NMR (δ) of CF₃CF₂CH₂CH₂CF₂CF₃: 11 (CF₃); 44.5 (CF₂); J_{H-F} was 16 Hz. ¹H NMR (δ): 2.5 m (2CH₂).
Mass spectrum (m/z, assignment, %): 247 (M-F, C₆H₄F₉⁺, 0.1); 227 (C₆H₃F₈⁺, 9.7); 197 (C₅H₄F₇⁺, 18.9); 177 (C₅H₃F₆⁺, 29.6); 157 (C₅H₂F₅⁺, 6.7); 127 (C₄H₃F₄⁺, 24.3); 119 (C₂F₅⁺, 5.6); 113 (C₃HF₄⁺, 31.3); 100 (C₂F₄⁺, 3.9); 77 (C₃H₃F₂⁺, 43.3); 69 (CF₃⁺, 68.4).
¹⁹F NMR (δ) of CF₃^{A}CF^{B}=CH²CH₂¹CF₂^{C}CF₃^{D}: -1.2 (3F^{a}); 10.5 (3F^{D}); 42.5 (2F^{C}); 55 (F^{B}); J(F^{A}-F^{B}) is 10 Hz. ¹H NMR (δ): 3.1 (2H¹); 5.8 (1H²); J(H²-F^{B}) is 30 Hz; J(H¹-H²) is 7.5 Hz; J(H¹-F^{C}) is 10 Hz. Raman spectrum (v, cm⁻¹): 1728.
Mast spectrum (m/z, assignment, %): 246 (M⁺, C₆H₃F₉⁺, 9.6); 227 (C₆H₃F₈⁺, 8.5); 207 (C₆H₂F₇⁺, 2.3); 177 (C₅H₃F₆⁺, 7); 157 (C₅H₂F₅⁺, 0.7); 127 (C₄H₃F₄⁺, 66); 119 (C₂F₅⁺, 2.6); 113 (C₃HF₄⁺, 20.3); 77 (C₃H₃F₂⁺, 100); 69 (CF₃⁺, 68.4). 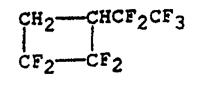 Mass spectrum (m/z, assignment, %): 227 (MF⁺, C₆H₃F₈⁺, 0.8); 207 (C₆H₂F₇⁺, 1.6); 177 (C₅H₃F₆⁺, 1.8); 157 (C₅H₂F₅⁺, 1.9); 127 (C₄H₃F₄⁺, 4.5); 113 (C₃HF₄⁺, 9.7); 100 (C₂F₄⁺, 13); 77 (C₃H₃F₂⁺, 12); 69 (CF₃⁺, 10.6); 64 (C₂H₂F₂⁺, 100).

### COMPARATIVE EXAMPLE C

### (CH₃)₂CHF + CF₂=CF₂ → Tar

Isopropyl fluoride (25 g), antimony pentafluoride (25 g) and tetrafluoroethylene (40 g) were added to a 400 mL Hastelloy™ nickel alloy shaker tube. The reaction mixture was agitated at 25°C for 12 hours. Only a tarry product was isolated.

### COMPARATIVE EXAMPLE D

### CF₃CH₂F + CF₂=CF₂ → PTFE

1,1,1,2-Tetrafluoroethane (50 g), antimony pentafluoride (5 g) and tetrafluoroethylene (40 g) were added to a 400 mL Hastelloy™ nickel alloy shaker tube. The reaction mixture was agitated at 50°C for 12 hours. The product isolated was polytetrafluoroethylene (PTFE, 10 g) along with recovered CF₃CH₂F (46 g).

### COMPARATIVE EXAMPLE E

### HCF₂CF₂CH₂F + CF₂ = CF₂ → PTFE

1,1,2,2,3-Pentafluoropropane (55 g), antimony pentafluoride (20 g) and tetrafluoroethylene (30 g) were added to a 400 mL Hastelloy™ nickel alloy shaker tube. The reaction mixture was agitated at 50°C for 12 hours. The product isolated was polytetrafluoroethylene (PTFE, 20 g) along with recovered HCF₂CF₂CH₂F (32 g).

### COMPARATIVE EXAMPLE F

### CH₂F₂ + CF₂=CF₂ → CH₂FCF₂CF₃ + CF₂=CF₂ → PTFE

Difluoromethane (55 g), antimony pentafluoride (20 g) and tetrafluoroethylene (TFE, 50 g) were added to a 400 mL Hastelloy™ nickel alloy shaker tube. The reaction mixture was agitated at 50°C for 12 hours. At this time addtional TFE (50 g) was added to the reaction mixture and agitation was continued for 12 hours at 50°C. The product isolated was polytetrafluoroethylene (PTFE, 20 g) along with recovered CF₃CF₂CH₂F (32 g).

## Claims

1. A process for forming an adduct of the formula RR¹R²CCR¹R²F or (FR¹R²CCRR²CH₂)₂ wherein R is selected from the group consisting of CH₃, CH₂F, C₂H₄F and F(CF₂)ₙCH₂CH₂ where n is an integer from 1 to 10, each R¹ is independently selected from the group consisting of H, Cl, F and CF₃ and each R² is independently selected from the group consisting of H, F and CF₃, comprising:
reacting a saturated compound of the formula RF with an olefin of the formula R¹R²C=CR¹R² in the liquid phase in the presence of antimony pentafluoride catalyst; provided that when (FR¹R²CCR¹R²CH₂)₂ is formed, the saturated compound is CH₃CHF₂ or FCH₂CH₂F and anhydrous HF is present.

2. A process according to Claim 1 wherein the saturated compound is CH₃F.

3. A process according to Claim 1 wherein the saturated compound is CH₂F₂.

4. A process according to Claim 1 wherein the saturated compound is CH₃CHF₂.

5. A process according to any one of Claims 1 through 4 wherein the olefin is CF₂=CF₂.

6. A process according to any one of Claims 1 through 4 wherein the olefin is CF₂=CFCF₃.

7. A process according to any one of Claims 1 through 4 wherein the olefin is CClF=CF₂.

8. A process according to any one of Claims 1 through 4 wherein the olefin is CHF=CF₂.

9. A process according to any one of Claims 1 through 4 wherein the olefin is CClF=CClF.

10. A process according to Claim 1, Claim 3, Claim 5, Claim 7 or Claim 9 wherein CH₂FCF₂CF₃ is formed.

## Patentansprüche

1. Verfahren zum Erzeugen eines Addukts der Formel RR¹R²CCR¹R²F oder (FR¹R²CCRR²CH₂)₂, wobei R aus der Gruppe, bestehend aus CH₃, CH₂F, C₂H₄F und F(CF₂)ₙCH₂CH₂, ausgewählt ist, wobei n eine ganze Zahl von 1 bis 10 ist, jedes R¹ unabhängig aus der Gruppe, bestehend aus H, Cl, F und CF₃, ausgewählt ist und jedes R² unabhängig aus der Gruppe, bestehend aus H, F und CF₃, ausgewählt ist, umfassend:
Umsetzen einer gesättigten Verbindung der Formel RF mit einem Olefin der Formel R¹R²C=CR¹R² in der flüssigen Phase in Anwesenheit von Antimonpentafluorid-Katalysator; mit der Maßgabe, daß, wenn (FR¹R²CCR¹R²CH₂)₂ erzeugt wird, die gesättigte Verbindung CH₃CHF₂ oder FCH₂CH₂F ist und wasserfreier HF vorhanden ist.

2. Verfahren nach Anspruch 1, wobei die gesättigte Verbindung CH₃F ist.

3. Verfahren nach Anspruch 1, wobei die gesättigte Verbindung CH₂F₂ ist.

4. Verfahren nach Anspruch 1, wobei die gesättigte Verbindung CH₃CHF₂ ist.

5. Verfahren nach einem der Ansprüche 1 bis 4, wobei das Olefin CF₂=CF₂ ist.

6. Verfahren nach einem der Ansprüche 1 bis 4, wobei das Olefin CF₂=CFCF₃ ist.

7. Verfahren nach einem der Ansprüche 1 bis 4, wobei das Olefin CClF=CF₂ ist.

8. Verfahren nach einem der Ansprüche 1 bis 4, wobei das Olefin CHF=CF₂ ist.

9. Verfahren nach einem der Ansprüche 1 bis 4, wobei das Olefin CClF=CClF ist.

10. Verfahren nach Anspruch 1, Anspruch 3, Anspruch 5, Anspruch 7 oder Anspruch 9, wobei CH₂FCF₂CF₃ erzeugt wird.

## Revendications

1. Procédé de formation d'un adduit de formule RR¹R²CCR¹R²F ou (FR¹R²CCRR²CH₂)₂ où R est choisi parmi le groupe constitué de CH₃, CH₂F, C₂H₄F et F(CF₂)ₙCH₂CH₂ où n est un entier de 1 à 10, chaque R¹ est indépendamment choisi parmi le groupe constitué de H, Cl, F et CF₃ et chaque R² est indépendamment choisi parmi le groupe constitué de H, F et CF₃, comprenant :
la réaction d'un composé saturé de formule RF avec une oléfine de formule R¹R²C=CR¹R² en phase liquide en présence de catalyseur de pentafluorure d'antimoine ; à condition que, quand (FR¹R²CCR¹R²CH₂)₂ est formé, le composé saturé soit CH₃CHF₂ ou FCH₂CH₂F et que HF anhydre soit présent.

2. Procédé selon la revendication 1 dans lequel le composé saturé est CH₃F.

3. Procédé selon la revendication 1 dans lequel le composé saturé est CH₂F₂.

4. Procédé selon la revendication 1 dans lequel le composé saturé est CH₃CHF₂.

5. Procédé selon l'une quelconque des revendications 1 à 4 dans lequel l'oléfine est CF₂=CF₂.

6. Procédé selon l'une quelconque des revendications 1 à 4 dans lequel l'oléfine est CF₂=CFCF₃.

7. Procédé selon l'une quelconque des revendications 1 à 4 dans lequel l'oléfine est CClF=CF₂.

8. Procédé selon l'une quelconque des revendications 1 à 4 dans lequel l'oléfine est CHF=CF₂.

9. Procédé selon l'une quelconque des revendications 1 à 4 dans lequel l'oléfine est CClF=CClF.

10. Procédé selon la revendication 1, la revendication 3, la revendication 5, la revendication 7 ou la revendication 9 dans lequel CH₂FCF₂CF₃ est formé.
